# EUROPEAN PATENT APPLICATION

(11) **EP 4 166 699 A1**
(43) Date of publication of application: **19.04.2023**
(21) Application number: 21202700.7
(22) Date of filing: 14.10.2021
(51) Int. Cl.: D01F 1/10, D01D 5/00, D04H 1/728, D06M 13/513, A61L 27/10, A61L 27/54

(54) **BIOCOMPATIBLE AND BIODEGRADABLE FIBROUS STRUCTURE CONTAINING SILICA-BASED SUBMICRON FIBERS, BIOGENIC IONS AND WITH A FUNCTIONAL SURFACE FOR BINDING ACTIVE SUBSTANCES AND A METHOD OF ITS PRODUCTION**

(71) Applicant: Technicka univerzita v Liberci, 460 01 Liberec 1 (CZ)
(72) Inventor: Rysova, Miroslava, 507 23 Detenice (CZ); Tomankova, Hana, 460 07 Liberec 3 (CZ)
(74) Representative: Musil, Dobroslav

(57) **Abstract**

The invention relates to a biocompatible and biodegradable fibrous structure containing silica-based submicron fibers, biogenic ions and with a functional surface for binding of active substances. The fibers are provided with bioactive ions and the fiber surface is functionalized with aminoalkoxysilane or epoxyalkoxysilane.

In addition, the invention relates to a method of producing the biocompatible and biodegradable fibrous structure according to claim 1 or 2, in which a sol prepared by a sol-gel method is spun. Before spinning, the sol is synthetized from low molecular weight tetraalkoxysilane precursor by the sol-gel method in alcohol with the addition of water under acid catalysis, wherein the sol is doped with bioactive ions in the form of salts already during its preparation before spinning and after spinning and the fiber surface is functionalized with aminoalkoxysilane or epoxyalkoxysilane.

## Description

### Technical field

The invention relates to a stable and at the same time biocompatible and biodegradable fibrous structure containing silica-based submicron fibers doped with bioactive ions or mixtures thereof with a surface bearing functional groups modifying the surface properties of the fibers and enabling possible binding of active substances to this surface.

The invention also relates to a method of producing these fibrous structures.

### Background art

Fibrous structures containing submicron fibers and nanofibers due to their unique properties - i.e. mainly small pore size combined with high porosity and small fiber diameter leading to extremely high specific surface area available for binding of molecules or for performing catalytic reactions, represent structures with high application potential in medicine and biotechnology. For the medical field, submicron fiber structures are particularly useful as carriers for tissue engineering of a variety of tissues and also as wound dressings. Here, the similarity of artificially prepared fibrous structures with naturally occurring structures of extracellular matrix (ECM) and/or membrane effect is used. The membrane effect stimulates the wound environment, promotes the so-called moist wound healing and at the same time allows gas exchange between the wound and the external environment. To achieve this effect, several conditions must be met, the most important of which is to maintain sufficient porosity in a humid environment.

Healing damaged tissue or growth of completely new tissue can also be promoted by the presence of signalling bioactive and biogenic molecules and ions which promote cell migration, differentiation or stimulation of the formation of selected ECM components. Simultaneously, these substances can also be used to decontaminate infected wounds or prevent postoperative infections. Such biogenic substances include, for example, silicon, whose presence in low concentrations enhances healing and stimulates collagen production. Furthermore, silver and copper ions have been described as having a high antimicrobial effect. Furthermore, silver and copper ions have been described as substances with a high antimicrobial effect.

In biotechnological applications, but also in medical applications, conjugation of active molecules to various substrates is used in order to improve their stability, reduce the load of the reaction medium with free active molecules and prolong their effective time when applied in situ. Here, it is possible to use a combination of bioactive ions and, for example, conjugated enzymes, whose catalytic activity can be directly dependent on or supported by the presence and gradual release of selected bioactive ions. Such an effect has been described, for example, in the case of calcium ions in relation to the activity of the enzyme trypsin or in the case of zinc in relation to the activity of digestive enzymes.

In this case, the subsequent functionalization of the surface leads to a change in the wettability of the surface by an aqueous solution, which consequently has two effects. Firstly, it increases the efficiency of the subsequent binding of the active molecule from aqueous buffers and, secondly, it leads to improvement in the release kinetics of incorporated ions supporting the activity of the bound molecule. This effect can also be supported by the application of physical methods during the production of a fibrous cover, which lead to improved surface wettability before the actual chemical functionalization (by functional group grafting) and increase the efficiency of this process and the number of functional groups per unit area.

CZ 2003-2421, or WO2005024101, discloses a method of producing nanofibers by electrostatic spinning, however, polymer solutions for fiber preparation are not specified.

CZ PV 2012-549 discloses a nanofibrous structure with an immobilized organic agent, which consists of pure silica nanofibers with a surface modified with aminoalkoxysilane and subsequently immobilized with an organic agent. This nanofibrous structure is made by forming pure silica nanofibers by electrostatic spinning from a starting sol synthetized by a sol-gel method from tetraalkoxysilane, whereby the nanofibers are afterwards heat treated and then the surface of the nanofibers is modified with an aminoalkoxysilane solution, whereupon the thus modified surface of the nanofibers is immobilized with organic agents.

CZ 2020-37178 describes the preparation of pure silica nanofibers with grafted functional groups and an immobilized organic agent. To increase the binding efficiency, plasma surface pre-treatment and subsequent chemical modification are combined here.

CZ 2015-406 discloses a method of producing hybrid nanofibrous structures with immobilized active agents, consisting in preparing a starting hybrid salt by a sol-gel method, namely by enriching the basic reaction mixture containing tetraalkoxysilane by a proportion of aminopropyltriethoxysilane, whereupon the hybrid sol thus prepared is subsequently spun and after that the nanofibrous structure formed is thermally stabilized under specific conditions, namely by exposure to temperatures of up to 200°C. Afterwards, the nanofibrous layer with an active surface thus formed is exposed to a solution for immobilization of the selected active substance, wherein the active substance is bound to the surface of the above-mentioned nanofibrous layer by peptide and/or hydrogen bonds. The technical solution also relates to a hybrid nanofibrous structure, formed as a nanofibrous spatial structure based on nanofibers activated by -NH₂ groups, wherein an active substance is bound to the surface of the nanofibers by peptide bonding or hydrogen (bridge) bonding, wherein it is preferably a hybrid nanofibrous layer produced by the method as described above, the active substance being preferably an active pharmaceutical ingredient or drug substance. However, in the nanofiber structures thus prepared, the availability of functional groups on the surface of the nanofibers for further binding of active agents is limited.

CZ PV 2012-549, CZ 2020-37178, CZ 2015-406 do not contain bioactive ions except silicon, which constitutes their basic building block, but whose release from the structure is not declared.

In document WO 2009018104 methyltrimethoxysilane is used as a precursor for the preparation of silica nanofibers. Without heat treatment of the nanofibers according to WO 2009018104 or at low temperatures of heat treatment of the nanofibers according to WO 209018104, these nanofibers have due to the presence of a methyl group on their surface hydrophobic properties and exhibit a low number of Si-OH groups on the surface required for any subsequent modification of the surface by aminoalkylalkoxysilane. For these reasons, the solution according to WO 2009018104 is not suitable for the preparation of starting silica nanofibers for the subsequent surface modification and possible immobilization of organic agents.

Documents JP 20040041335, JP 20040161234 and JP 20040243580 describe the preparation of an organic-inorganic nanofibrous composite composed of a regular skeleton of polyethyleneimide fibers with silica layers deposited using the sol-gel method. The resulting composite material is to serve to capture or concentrate various substances in the prepared structure, but the capture of the desired particles occurs only as it is in the case of a filter, i.e., in the gaps between individual nanofibers, or by simple adsorption of the desired particles to the volume of the nanofibrous composite.

Wrapping paper according to KR 2090058155 is made of nanofibers which were obtained by electrostatic spinning of a biodegradable organic polymer with the addition of a sol of silicon dioxide and silver nitrate. The resulting product has antiseptic and antibacterial properties, but it does not exhibit a functional surface and is not adapted to immobilize organic agents.

KR 20100058372 discloses the preparation of a catalyst from mesoporous silica nanofibers prepared by growth from a gas phase and subsequent introduction of the catalyst using silane onto the surface and into the pores of the fibers thus formed. The resulting product is claimed as a catalyst for various organic reactions, but it does not contain biogenic ions, nor does it serve to immobilize organic agents.

Utility model CZ 2019-37061 describes a wound dressing with a nanofibrous layer for drug delivery, wherein the active substance is bound in carrier nanoparticles - liposomes, for which the nanofibrous structure acts as a support system enabling their application at the wound site. Nanofibers prepared by electrostatic spinning of organosilicate can also be used here as support nanofibers. These fibers are not otherwise surface-functionalised. The disadvantage of this solution is the impossibility of binding additional active substances.

Patent document CZ PV2012-166 and utility model CZ 31723 disclose biocompatible fibrous (nanofibrous and microfibrous) structures useful for the treatment of wounds and skin defects. In the case of CZ 31723 the structure based on copolymers of L-lactide and ε-caprotactone does not contain functional groups for binding agents on its surface, nor does it contain bioactive ions. In the case of CZ PV2012-166, the disadvantages of the carboxymethyl cellulose-based material include low stability in an aqueous medium, the absence of active substances/ions and limited surface functionality.

Solutions with a content of ions are also known, as well as solutions presenting conjugation of enzymes to polymeric nanofibers.

Patent document EP 3 042 628 A1 discloses bioglass-based fiber dental implants. However, they do not contain submicron-diameter surface functionalized fibers and their structure is mainly based on the content of phosphate components, such as tricalcium phosphate and others. Patent document WO2007017756A2 discloses a solution for the preparation of bioglass-based scaffolds. However, these are prepared through the polymer template-assisted layer-by-layer method and do not contain submicron fibers. The situation is the same for other bioglasses, although they contain bioactive ions, but their structure contains other components and is not surface functional and fibrous.

The disadvantage of the background art is the absence of a comprehensive solution that would simultaneously allow the use of the effect of bioactive ions and of the possibility of surface immobilization of active substances in sufficient quantities and the achievement of their synergistic effect.

The objective of this invention is to eliminate or at least minimize the disadvantages of the background art.

### Principle of the invention

The objective of the invention is achieved by a silica fibrous structure with a fiber diameter on a submicron scale, which is doped with bioactive ions and at the same time is surface functionalized so as to achieve 2D or 3D structure with increased antibacterial activity or other biological activity and at the same time to allow the application of the fibrous structure in this form or its further use for the conjugation of molecules to their surface in sufficient quantities due to the modification of the surface with aminoalkylalkoxysilane or epoxyalkoxysilane. Such modification leads to an increase in surface wettability while allowing electrostatic bonding, hydrogen bonding or covalent bonding depending on the use of conjugation chemistry, whereby the efficiency of the modification can be increased by adding physical activation of the fiber surface by plasma treatment.

Silica submicron fibers are used as a carrier substrate in the present invention due to their biocompatibility, the unique method of biodegradation, as well as due to the wide possibilities of their surface modifications. It has been proved that silica-based nanostructures are not only biocompatible with different tissue types, but also bioactive due to the release of orthosilicic acid during their degradation. The degradation takes place by gradual erosion of the surface, whereby there is no reduction in the porosity of the layer due to the swelling of the individual fibers. Another indisputable advantage of silica fibers is the high proportion of silanol Si-OH groups on their surface, which can be used to modify their surface by functional alkoxysilane covalent bonding. The frequency of the resulting functional groups incorporated on the surface is then modified by the reaction conditions and can also be increased by pretreatment of the fiber surface prior to binding.

The principle of the production of the submicrofibrous structure consists in the preparation of a spinning sol. The spinning sol is prepared by the sol-gel method from tetraalkoxysilane by acid catalysis in alcohol, but without the addition of another auxiliary polymer, and during the preparation process of the preparation, this sol is doped with selected bioactive ions in the form of suitable compounds - most often nitrates. The sol thus prepared is subsequently processed into non-woven fabric with fiber diameters on a sub-micron scale. Depending on the parameters of the spinning sol and the spinning conditions, it is even possible to achieve structures with a majority of nanofibers (i.e., fibers with diameters ≤100 nm) in the structure. The production of the fibrous structure can be performed by both the electrostatic spinning method and centrifugal spinning method. The formed fibrous structure is then thermally stabilized and optionally treated with a suitable type of plasma. This step increases the efficiency of surface functionalization with alkoxysilane, but it is not necessary. Subsequently, the surface is modified with the selected aminoalkoxysilane or epoxyalkoxysilane. Thus, a biocompatible and bioactive fibrous layer is obtained with a functional surface available for binding the active substance to its surface via the chosen conjugation chemistry.

In the preparation of the spinning sol doped with individual bioactive ions (Ca²⁺, Zn ²⁺, Ag⁺, Cu²⁺) or with a combination thereof, the spinning sol being formed by the sol-gel method from tetraalkoxysilane involving acid catalysis, the following ratios of starting materials must be observed.

In the case of the molar ratio of tetraalkoxysilane to water, it is maintained in the range of 0.3 to 0.65 and at the same time the molar ratio of acid to tetraalkoxysilane is maintained in the range of 0.005 to 1. When doping with bioactive ions, it is then necessary to maintain the molar ratio of sol to tetraalkoxysilane in the range of 0.001 to 0.25. After completion of polycondensation reaction, the concentration of the spinning sol is adjusted to the range of 25 to 45 %. By this process, spinning sol is prepared which can be processed to achieve a fiber structure with a fiber diameter on the submicron scale. The structure of the prepared layer is determined by the chosen spinning method, where the use of electrostatic spinning leads to the preparation of mostly 2D structures, while the use of centrifugal spinning allows the formation of 3D structures with a higher proportion and dimensions of interfiber pores. The diameter of the fibers in the layer and the thickness of the spun layer are determined by the spinning conditions. Most often, the basis weight is in the range of 10-50 g/m², but these limits are not restrictive. The prepared structures are further thermally treated to achieve a higher mechanical stability. Thermal stabilization most often takes place at temperatures of up to 350 °C, but other modifications can be carried out without thermal treatment. Similarly, the layer can be hydrophilized by physical treatment with low pressure plasma or with plasma treatment conducted at atmospheric pressure before the functional groups are bonded. This treatment leads to an increase in the number of Si-OH groups on the surface, whereby a consequently higher surface functionality is achieved. Plasma treatment can also be used for fibers stabilized at temperatures above 350 °C to re-induce Si-OH groups lost due to thermal treatment performed in order to achieve higher mechanical resistance of the layer. At higher temperatures, there is no loss of biocompatibility in silica nanofibers. This was confirmed for fibers treated at temperatures of up to 750 °C.

Subsequently, the surface of the doped silica fibers is functionalized by covalent bonding of functional alkoxysilane in the form of aminoalkoxysilane or epoxyalkoxysilane in concentrations most often in the range of 0.1 to 5 % in the solution, but possibly also in the range of 0.1 to 75 %, more preferably in the range of 0.1 to 60 %, even more preferably in the range of 0.1 to 25 % and most preferably in the above-mentioned range of 0.1 to 5 %. The surface functionalization does not reduce the biocompatibility of the surface but modifies its charge and wettability. This modification leads to an improvement in the availability of active ions incorporated into the fiber mass and at the same time can be used for adsorption, electrostatic or covalent binding of other molecules to the fiber surface depending on the specific biotechnological or medical application chosen, so as to achieve, if possible, a synergistic effect with the incorporated ions. These two types of functional groups on the surface allow the binding of most active molecules using spontaneous binding or via auxiliary conjugation chemistry. An example of such chemistry may be the use of various types of the so-called zero-length conjugation linkers based on ethyl (dimethylaminopropyl) carbodiimide.

### Description of the drawings

The invention is schematically illustrated in the drawings, where Fig. 1 shows SEM microscopic morphology of silica-based submicron fibers containing bioactive zinc ions according to Example 1, Fig. 1a shows the representation of individual classes of fiber diameters in a layer according to Example 1, Fig. 2 shows evidence of biocompatibility of silica-based submicron fibers containing bioactive zinc ions according to Example 1 before and after surface functionalization, evaluated on HaCaT cells, Fig. 3 shows SEM microscopic morphology of silica-based submicron fibers containing bioactive copper ions according to Example 2, Fig. 3a shows the representation of individual classes of fiber diameters in a layer according to Example 3, Fig. 4 shows evidence of surface functionality and biocompatibility of silica-based submicron fibers containing bioactive copper ions according to Example 2 before and after surface functionalization, evidence of the presence of amino groups on the surface of untreated and treated fibers by methyl orange staining, biocompatibility before and after surface functionalization, evaluated on HaCaT cells, Fig. 5 shows SEM microscopic morphology of silica-based submicron fibers containing bioactive calcium ions according to Example 3, Fig. 5a shows the representation of individual classes of fiber diameters in a layer according to Example 3, Fig. 6 shows SEM microscopic morphology of silica-based submicron fibers containing a mixture of bioactive silver, copper and zinc ions according to Example 4, Fig. 6a shows the representation of individual classes of fiber diameters in a layer according to Example 4, Fig. 7 shows evidence of biocompatibility of silica-based submicron fibers containing a mixture of bioactive silver, copper and zinc ions according to Example 4 before and after surface functionalization, evaluated on HaCaT cells, and Fig. 8 shows evidence of antibacterial activity of silica-based submicron fibers containing a mixture of bioactive ions against bacteria S. gallinarum (on the left-hand side) and E. coli (on the right-hand side).

### Examples of embodiment

The invention will be described using examples of the formation of a silica-based fibrous structure doped with biogenic ions and with a modified surface, which further allows binding of active substances and active pharmaceutical ingredients and other molecules selected according to the specific application. The invention is illustrated by selected specific embodiments, by which, however, not all possible embodiments of the invention are described. These possibilities, which are not described in more detail here, will be apparent to a person skilled in the art from this text without the need for further inventive activity.

### Example 1

A spinning sol was prepared by a modified sol-gel method by mixing 164 ml of isopropyl alcohol and 200 ml of tetraethoxysilane, water and acid so that the molar ratios corresponded to 0.45 molar ratio of tetraethoxysilane to water and at the same time to 0.1 molar ratio of acid to tetraethoxysilane. Simultaneously, zinc nitrate was added as a source of bioactive ions at a ratio of 0.01 moles to tetraethoxysilane. The sol thus prepared underwent the processes of hydrolysis, polycondensation and maturation, in which it is finally concentrated to 33 % of the dry matter content of the total weight of the spinning sol. This step was accomplished by evaporating the solvent.

The spinning sol thus prepared was subsequently processed by the method of electrostatic spinning using direct current (the so-called DC electrospinning) from a wire electrode while maintaining the spinning conditions at a spinning distance of 160 mm and electrode voltage difference of 60 kV. A fiber layer with a mean fiber diameter of 174 nm was prepared by this method, whereby the basis weight of the fibers was determined by the speed of movement of the substrate material of 12 g/m². To achieve higher layer cohesion, temperature stabilization was performed by exposure to a temperature of 180 ° C for a period of 2 hours. This heat treatment did not lead to a significant change in the structure (homogeneity) of the layer or the diameters of the submicron fibers. To achieve surface functionality, the layer was then wetted in a 3% solution of 3-aminopropyltryethoxysilane dissolved in 96% ethanol for 2 hours at laboratory temperature. The stability of the functional groups grafted on the surface was enhanced after rinsing excess functionalizing agent, performed by repeated rinsing in ethanol solution, by heat treatment at a temperature of 110 ° C for a period of 30 minutes. By this method were obtained silica nanofibers containing bioactive zinc and at the same time having a functional surface containing -NH₂ groups available for further binding by the chosen method.

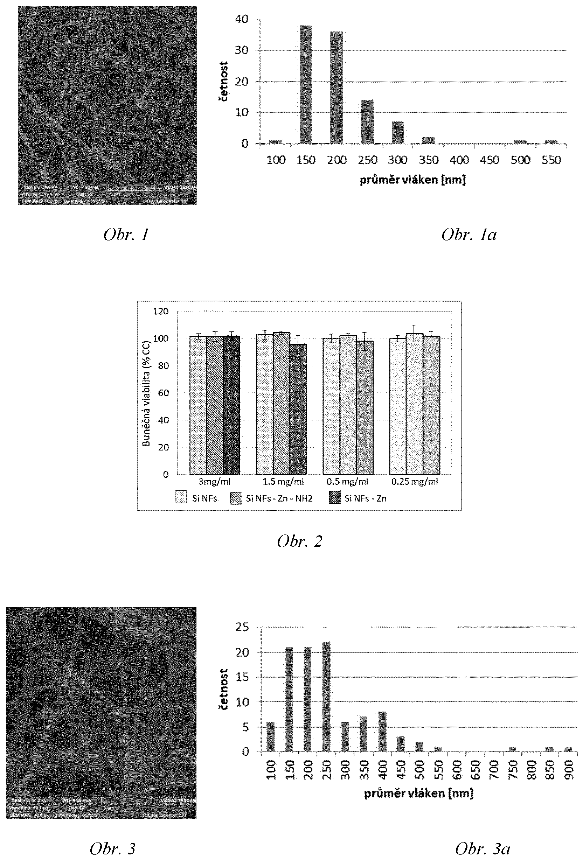

The morphology of the thus prepared submicron fibers is shown in Fig. 1 taken by electron microscopy, and the representation of the different fiber diameter classes in the layer according to Example 1 is shown in Fig. 1a. The material thus prepared was proved to be biocompatible with skin cover cells. The in vitro test on human HaCaT keratinocytes was performed in accordance with the standard CSN EN ISO 10993-5:2009 by preparing eluate of the test sample in various concentrations by elution for a period of 24 hours. The eluate was then exposed to precultured cells for another 24 hours. The resulting cytocompatibility was assessed by the metabolic activity of living cells with the tetrazolium salt of WST-8 (2- (2-methoxy-4-nitrophenyl) -3- (4-nitrophenyl) -5-(2,4-disulfophenyl) -2H-tetrazolium, sodium salt) and by subsequent quantification at a wavelength of 450 nm. The results obtained in the form of evidence of biocompatibility of silica-based submicron fibers according to Example 1 before and after surface functionalization, evaluated on HaCaT cells, are shown in Figure 2.

This layer can be applied in this state without further modification, or it can be used to further bind biotechnologically or medically important substances. In this case, for example, it can be used as a carrier for a biotechnologically usable laccase enzyme for water treatment.

### Example 2

A spinning sol was prepared by a modified sol-gel method by mixing 164 ml of isopropyl alcohol and 200 ml of tetraetylorthosilicate (tetraethoxysilane), water and acid so that the molar ratios corresponded to 0.4 molar ratio of tetraethoxysilane to water and at the same time to 0.11 molar ratio of acid to tetraethoxysilane. Simultaneously, copper nitrate was added as a source of bioactive ions at a ratio of 0.034 moles to tetraethoxysilane. The sol thus prepared underwent the processes of hydrolysis, polycondensation and maturation, and finally was concentrated to 35 % of the dry matter content of the total weight of the spinning sol. This step was accomplished by evaporating the solvent.

The spinning sol thus prepared was subsequently processed by the method of electrostatic spinning using direct current (the so-called DC electrospinning) from a rod electrode while maintaining the spinning conditions at a spinning distance of 150 mm and electrode voltage difference of 45kV. A fiber layer with a mean fiber diameter of 289 nm was prepared by this method. To achieve higher layer cohesion, temperature stabilization was performed by exposure to a temperature of 180 °C for a period of 2 hours. To increase the efficiency of surface functionalization, the fibrous layer was subsequently treated by corona discharge in air atmosphere at room temperature, at a pressure of -101 kPa, power of 800 W and material movement speed of 1 m/min. The activated layer was subsequently transferred to a functionalization bath consisting of a 3% solution of 3-aminopropytryethoxysilane dissolved in 96% ethanol, where it was wetted for a period of 1 hour at laboratory temperature. The stability of the functional groups grafted onto the surface was enhanced after rinsing off the excess functionalizing agent, performed repeatedly in the ethanol solution, by heat treatment at a temperature of 110 °C for a period of 30 minutes. By this method were obtained silica nanofibers containing bioactive copper ions and at the same time having a functional surface containing -NH₂ groups available for further binding by the chosen method.

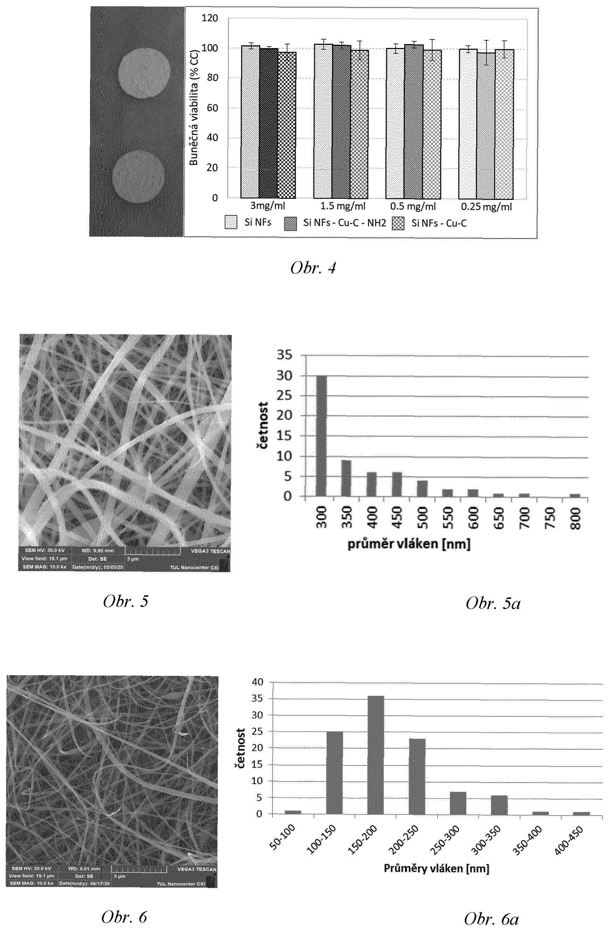

The morphology of the thus prepared submicron fibers is shown in Fig. 3 taken by electron microscopy, and the representation of different fiber diameter classes in the layer according to Example 3 is shown in Fig. 3a. The material thus prepared was proved to be biocompatible with skin cover cells. The in vitro test on human HaCaT keratinocytes was performed in accordance with the standard CSN EN ISO 10993-5:2009 in such a manner that eluate of the test sample in various concentrations was prepared by elution for a period of 24 hours. The eluate was then exposed to precultured cells for another 24 hours. The resulting cytocompatibility was assessed by the metabolic activity of living cells with the tetrazolium salt of WST-8 (2- (2-methoxy-4-nitrophenyl) -3- (4-nitrophenyl) -5- (2,4-disulfophenyl) -2H-tetrazolium, sodium salt) and by subsequent quantification at a wavelength of 450 nm. The viability of the exposed cells at all concentrations exceeded 95% of the viability of the cell control. At the same time, the surface treatment resulted in a slight increase in cell viability compared to the untreated doped layer. The results achieved, in the form of the evidence of surface functionality and biocompatibility of silica-based submicron fibers containing bioactive copper ions according to Example 2 before and after surface functionalization, the evidence of the presence of amino groups, untreated on the surface and treated on the surface by methyl orange staining, biocompatibility before and after surface treatment assessed on HaCaT cells, are shown Fig. 4.

This layer can be applied in this state without further modification, or it can be used for further binding of biotechnologically or medically important substances. In this case, e.g., a combination of an active silica layer and natural antiseptics, growth factors or other substances supporting angiogenesis and wound healing is suitable. This can be bonded by simple adsorption or electrostatic bonding or covalent bonding while maintaining a high porosity of the silica layer. In the case of protein substances, their presence on the surface can be demonstrated by (non-)specific staining of proteins and their quantification.

### Example 3

A spinning sol was prepared by a modified sol-gel method by mixing 164 ml of isopropyl alcohol and 200 ml of tetraethylorthosilicate (tetraethoxysilane), water and acid so that the molar ratios corresponded to 0.43 molar ratio of tetraethoxysilane to water and at the same time to 0.1 molar ratio of acid to tetraethoxysilane. Simultaneously, calcium nitrate was added to the mixture as a source of bioactive ions at a ratio of 0.0072 moles to tetraethoxysilane. The sol thus prepared underwent the processes of hydrolysis, polycondensation and maturation, when it was finally concentrated to 29 % of the dry matter content of the total weight of the spinning sol. This step was accomplished by evaporating the solvent.

The spinning sol thus prepared was subsequently processed by the method of centrifugal spinning while maintaining the spinning conditions at 200 mm and 9000 RPM. A three-dimensional fiber layer with a mean fiber diameter of 463 nm was prepared by this method. The morphology of the thus prepared submicron fibers is shown in Fig. 5, and the representation of different fiber diameter classes in the layer according to Example 3 is shown in Fig. 5a. To achieve higher layer cohesion, temperature stabilization was performed by exposure to a temperature of 180 °C for a period of 2 hours. Subsequently, the layer was surface functionalized by wetting for a period of 30 minutes in a 2% mixture of 3-glycidyloxypropyltriethoxysilane and ethanol with a 15% proportion of water. After repeated rinsing in ethanol solution, the layer was dried at room temperature for a period of 48 hours. By this method were obtained silica nanofibers containing bioactive calcium ions and at the same time having a functional surface containing epoxy groups available for further binding by the chosen method.

This layer can be applied in this state without further modification and can be used for in situ conjugation (e.g., as a sensor). Or it can be conjugated to biotechnologically or medically important substances to ensure their increased stability during subsequent application. This surface functionalization ensures reactivity with a full spectrum of substances including amino groups, sulfanyl groups and others. In this case, a combination of the active silica layer and the conjugated enzyme trypsin used for wound debridement is suitable.

### Example 4

A spinning sol was prepared by a modified sol-gel method by mixing 164 ml of isopropyl alcohol and 200 ml of tetraetylorthosilicate (tetraethoxysilane), water and acid so that the molar ratios corresponded to 0.37 molar ratio of tetraethoxysilane to water and at the same time to 0.11 molar ratio of acid to tetraethoxysilane. Simultaneously, silver nitrate, copper nitrate and zinc nitrate were added to the mixture as sources of bioactive ions at a ratio of 0.007/0.004/0.003 moles to tetraethoxysilane. The sol thus prepared underwent the processes of hydrolysis, polycondensation and maturation, and subsequently was concentrated to 36 % of the dry matter content of the total weight of the spinning sol. This step was accomplished by evaporating the solvent.

The thus prepared spinning sol was subsequently processed by the method of electrostatic spinning using direct current (DC electrospinning) from a wire electrode while maintaining the spinning conditions at a spinning distance of 165 mm and electrode voltage difference of 65 kV. A fiber layer with a mean fiber diameter of 193 nm was prepared by this method. To achieve higher layer cohesion, temperature stabilization was performed by exposure to a temperature of 200 °C for a period of 120 minutes. To achieve surface functionality, the layer was then wetted in a 5% solution of 3-aminopropyltryethoxysilane dissolved in 96% ethanol at laboratory temperature for a period of 30 minutes. The stability of the functional groups grafted on the surface after rinsing excess functionalizing agent performed by repeated rinsing in ethanol solution was enhanced by heat treatment at a temperature of 110 °C for a period of 30 minutes. By this method were obtained silica nanofibers containing bioactive silver, copper and zinc ions and at the same time having a functional surface containing -NH₂ groups available for further binding by the chosen method.

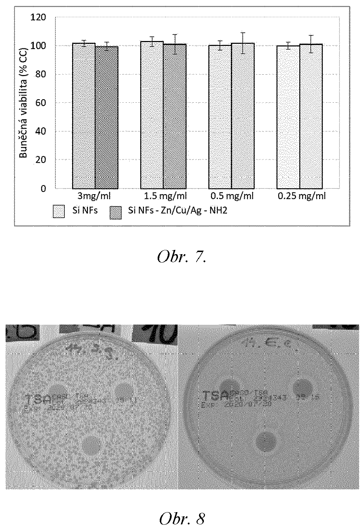

The morphology of the thus prepared submicron fibers is shown in Fig. 6 taken by electron microscopy, and the representation of different fiber diameter classes in the layer according to Example 4 is shown in Fig. 6a. The material thus prepared was proved to be biocompatible with skin cover cells according to the methodology described in Example 1. The results achieved are shown in Fig. 7 in the form of evidence of biocompatibility of silica-based submicron fibers containing a mixture of bioactive silver, copper and zinc ions according to Example 4 before and after surface functionalization, evaluated on HaCaT cells. Furthermore, the antibacterial activity of the layer against two model bacterial strains of Staphylococcus gallinarum and Escherichia coli was confirmed by a diffusion method. During the test, a bacterial suspension at a concentration of 10⁴ bacteria/ml was inoculated onto a culture agar plate. Subsequently, a triplicate of the tested samples was attached. After incubation under standard conditions (37 °C) for a period of 24 hours, the size of the diffusion zone around the samples was evaluated. The average size of 14 mm confirmed the antibacterial activity of the samples on both bacterial strains, as shown in Fig. 8 in the form of evidence of antibacterial activity of silica-based submicron fibers containing a mixture of bioactive ions against S. gallinarum (on the left-hand side) and E. coli (on the right-hand side).

This layer can be applied in this state without further modification and promote, for example, the cleaning and healing of contaminated and hard-to-heal wounds, or it can be used for further binding of biotechnologically or medically important substances. In this case, a combination with an antiseptic or antibiotic important for dermatovenereology is suitable. This can be bonded by simple adsorption or electrostatic bonding, while maintaining a high porosity of the silica layer. By this method, an active layer with a synergistic effect can be obtained combining an antibacterial surfactant released in the order of hours to days and bioactive ions providing protection against secondary infection after drug release from the surface or infection caused by microorganisms outside the surfactant area of action.

## Claims

1. A biocompatible and biodegradable fibrous structure with silica-based submicron fibers, biogenic ions and functional surface for binding active substances, **characterized in that** the fibers are provided with bioactive ions and the fiber surface is functionalized with aminoalkoxysilane or epoxyalkoxysilane.

2. The biocompatible and biodegradable fibrous structure according to claim 1, **characterized in that** the bioactive ions are calcium and/or copper and/or zinc and/or silver ions.

3. A method of producing the biocompatible and biodegradable fibrous structure according to claim 1 or 2, in which a sol prepared by a sol-gel method is spun, **characterized in that** before spinning, the sol is synthetized from low molecular weight tetraalkoxysilane precursor by the sol-gel method in alcohol with the addition of water under acid catalysis, the sol being doped with bioactive ions in the form of salts already during its preparation before spinning and after spinning, the fiber surface is functionalized with aminoalkoxysilane or epoxyalkoxysilane.

4. The method according to claim 3 **characterized in that** before spinning, the sol is synthesized in a solution of ethanol or isopropyl alcohol and the molar ratio of tetraalkoxysilane to water is in in the range of 0.3 to 0.65, whereby the pH of the sol during the reaction is adjusted by the addition of an inorganic or organic acid at a ratio of acid to tetraalkoxysilane in the range of 0.005 to 1.

5. The method according to claim 3 or 4, **characterized in that** excess alcohol is removed before spinning the sol and the final dry matter concentration in the solution is in the range of 25 to 45 %.

6. The method according to any of claims 3 to 5, **characterized in that** the sol is doped with bioactive calcium and/or copper and/or zinc and/or silver ions while maintaining the molar ratio of the salt to tetraalkoxysilane in the range of 0.001 to 0.25.

7. The method according to any of claims 3 to 6, **characterized in that** spinning is performed by electrostatic and/or centrifugal spinning, whereupon the fibrous structure is thermally treated and, if necessary, it is also treated with plasma to increase the efficiency of the subsequent surface modification with the selected functional alkoxysilane.

8. The method according to any of claims 3 to 7, **characterized in that** the fiber surface is functionalized in water, alcohol or another organic solvent for a period of 0.5 to 12 hours at a concentration of functional alkoxysilane of 0.1 to 75 %, preferably 0.1 to 60 %, more preferably 0.1 to 25 % and ideally 0.1-5 %.
